# EUROPÄISCHE PATENTANMELDUNG

(11) **EP 4 813 438 A2**
(43) Veröffentlichungstag der Anmeldung: **30.09.2026**
(21) Anmeldenummer: 26198966.9
(22) Anmeldetag: 19.07.2022
(51) Int. Cl.: A61M 37/00

(54) **APPLIKATIONSVORRICHTUNG FÜR MINDESTENS EIN IMPLANTAT**

(30) Priorität: 03.08.2021 DE 102021120149
(62) Dokumentnummer(n) der früheren Anmeldung(en) nach Art. 76 EPÜ: 22754807.0 / 4 380 666
(71) Anmelder: AMW GmbH, 83627 Warngau (DE)
(72) Erfinder: Barth, Dirk, 82166 Gräfelfing (DE); Feuersinger, Alexandra, 83059 Kolbermoor (DE); Kaffl, Hubert, 83730 Fischbachau (DE); Oliv, Lukas, 83700 Rottach-Egern (DE); Rodler, Stefanie, 83626 Valley (DE); Wiedenbauer, Magnus, 83714 Miesbach (DE); Hubner, Alexander, 95236 Stammbach (DE); Paech, Bernd, 91325 Adelsdorf (DE); Schnabel, Frank, 95183 Feilitzsch (DE); Tittel, Andreas, 07907 Oettersdorf (DE); Villa, Andrea, 95355 Presseck (DE)
(74) Vertreter: Beckord & Niedlich Patentanwälte PartG mbB

(57) **Zusammenfassung**

Eine Applikationsvorrichtung (1) für mindestens ein Implantat hat eine Kanüle (3), über die das Implantat einem Implantations-Zielort zugeführt wird. Ein Applikator-Gehäusekörper (32) hat ein Depotvolumen zur Bevorratung des Implantats. Ein Kolben (11) einer Kolbeneinrichtung (10) ist zwischen einer Ausgangsstellung und einer Implantationsstellung verlagerbar. Die Kolbeneinrichtung (10) hat einen Kolben-Betätigungskörper (12), mit dem der Kolben (11) fest verbunden ist. Der Applikator-Gehäusekörper (32) dient dabei zum Führen einer Verlagerungsbewegung der Kolbeneinrichtung (10). Zur überwindbaren Verriegelung des Applikator-Gehäusekörpers (32) mit der Kolbeneinrichtung (10) in deren Ausgangsstellung dient eine Ausgangsstellungs-Verriegelungseinrichtung. Diese ist umstellbar zwischen einer Verriegelungsstellung und einer Funktionsstellung, in der die Kolbeneinrichtung (10) zur Verlagerung des Kolbens (11) relativ zum Applikator-Gehäusekörper (32) verlagerbar ist. Es resultiert eine Applikationsvorrichtung (1), deren Funktionssicherheit bei gleichzeitig einfachem und Massenherstellungstechniken erreichbarem Aufbau verbessert ist.

## Beschreibung

Die Erfindung betrifft eine Applikationsvorrichtung für mindestens ein Implantat.

Eine derartige Applikationsvorrichtung ist bekannt aus der EP 3 456 374 B1.

Es ist eine Aufgabe der vorliegenden Erfindung, eine Applikationsvorrichtung für mindestens ein Implantat derart weiterzubilden, dass deren Funktionssicherheit bei gleichzeitig einfachem und mit Massenherstellungstechniken erreichbarem Aufbau verbessert ist.

Diese Aufgabe ist erfindungsgemäß gelöst durch eine Applikationsvorrichtung mit den im Anspruch 1 angegebenen Merkmalen.

Eine erfindungsgemäße Applikationsvorrichtung für mindestens ein Implantat weist eine Kanüle, über die das Implantat einem Implantations-Zielort zugeführt wird, einen Applikator-Gehäusekörper mit einem Depotvolumen und eine Kolbeneinrichtung mit einem Kolben auf.

Das "Depotvolumen" ist ein zur Bevorratung des Implantats vorgesehener, fest definierter Volumenbereich innerhalb der Applikationsvorrichtung, der sich vorzugsweise in einem Bereich des Applikator-Gehäusekörpers vor der Kanüle befindet. Das Depotvolumen könnte aber grundsätzlich auch einen Bereich in der Kanüle (mit-)umfassen, z. B. ein hinteres Ende der Kanüle an oder in dem Applikator-Gehäusekörper.

Der Kolben ist zwischen einer "Ausgangsstellung", in der ein Kolben-Ausstoßende des Kolbens auf einer von einer freien Kanülenspitze abgewandten Seite des Depotvolumens liegt, und einer "Implantationsstellung" verlagerbar, in der ein Kolben-Ausstoßende, also eine zum Implantat weisende Stirnseite des Kolbens, im Bereich einer freien Kanülenspitze liegt. In der Ausgangsstellung kann sich also ein zu implantierendes Implantat noch im vorgesehenen Depotvolumen befinden, da der Kolben noch nicht in das Depotvolumen hineinragt.

Dass sich das Kolben-Ausstoßende "im Bereich einer freien Kanülenspitze" befindet, ist dagegen so zu verstehen, dass das Kolben-Ausstoßende zumindest so weit nach vorne in die Kanüle verlagert ist, dass das Implantat in der Kanüle positioniert ist (vorzugsweise in einem kurzen Abstand hinter der Kanülenspitze), wobei sich das Implantat dann schon am Implantations-Zielort befinden kann, wenn die Kanüle ordnungsgemäß in das Gewebe eingestochen ist, aber noch von der Kanüle umgeben ist. Wenn die Kanüle nachfolgend wieder aus dem Gewebe herausgezogen wird, kann z. B. durch geeignete weitere Verlagerung des Kolbens relativ zur Kanüle dafür gesorgt werden, dass das Implantat am Implantations-Zielort im Gewebe verbleibt, wie später noch näher erläutert wird.

Die Kolbeneinrichtung weist hierzu einen Kolben-Betätigungskörper auf, mit dem der Kolben verbunden ist. Die Verbindung zwischen Kolben und Kolben-Betätigungskörper kann lösbar ausgebildet sein, d. h. der Kolben ist beispielsweise im Kolben-Betätigungskörper eingeschraubt oder lösbar eingeklemmt. Vorzugsweise handelt es sich aber um eine feste Verbindung. Der Applikator-Gehäusekörper selbst ist zum Führen einer Verlagerungsbewegung der Kolbeneinrichtung zwischen der Ausgangsstellung und der Implantationsstellung ausgebildet.

Erfindungsgemäß weist die Applikationsvorrichtung eine Ausgangsstellungs-Verriegelungseinrichtung zur überwindbaren Verriegelung des Applikator-Gehäusekörpers mit der Kolbeneinrichtung in deren Ausgangsstellung auf. Diese Ausgangsstellungs-Verriegelungseinrichtung ist zwischen einer Verriegelungsstellung, in der die Kolbeneinrichtung am Applikator-Gehäusekörper in der Ausgangsstellung fixiert ist, und einer Funktionsstellung umstellbar, in der die Kolbeneinrichtung zur Verlagerung des Kolbens relativ zum Applikator-Gehäusekörper verlagerbar ist.

Über die Ausgangsstellungs-Verriegelungseinrichtung ist funktionssicher erreicht, dass die Applikationsvorrichtung nicht unerwünscht ausgelöst wird, dass also die Kolbeneinrichtung unerwünscht von der Ausgangsstellung in Richtung der Implantationsstellung verlagert wird. Die Ausgangsstellungs-Verriegelungseinrichtung kann als Rasteinrichtung mit mindestens einer Rastnase und/oder mit mindestens einer Rastöffnung und/oder mit mindestens einem Rasthaken ausgeführt sein.

Die Ausgangsstellungs-Verriegelungseinrichtung kann insbesondere zwei verschiedene Typen von Rastnasen aufweisen, die mit unterschiedlichen Gegenkörpern zusammenwirken. Komponenten der Verriegelungseinrichtung können einerseits an der Kolbeneinrichtung, insbesondere an einem Kolben-Betätigungskörper, und andererseits an einem Applikator-Gehäusekörper ausgeführt sein.

Hauptkomponenten der Applikationsvorrichtung können als Kunststoffkomponenten und insbesondere als Spritzgusskomponenten ausgeführt sein. Alle Komponenten der Applikationsvorrichtung können als Kunststoffkomponenten ausgeführt sein. Vorzugsweise sind die Kanüle und der Kolben jedoch aus Edelstahl.

Der Kolben kann bevorzugt fest mit dem Kolben-Betätigungskörper verbunden sein.

Des Weiteren betrifft die vorliegende Erfindung die humanmedizinische, veterinärmedizinische oder kosmetische Verwendung der erfindungsgemäßen Applikationsvorrichtung.

Bevorzugt erfolgt die Verwendung der erfindungsgemäßen Applikationsvorrichtung zur therapeutischen Anwendung, besonders bevorzugt zur Verabreichung von Arzneistoffen, insbesondere zur Verabreichung von (synthetischen) Analoga des Neurohormons Gonadotropin-Releasing-Hormon (GnRH), insbesondere bevorzugt zur Behandlung von Prostata-oder Brustkrebs, Endometriose, Uterusmyom, Endometriumablation, verfrüht einsetzender Pubertät (Pubertas praecox), Transsexualität, Cluster-Kopfschmerz und in der humanmedizinischen und veterinärmedizinischen Reproduktionsmedizin.

Weitere, besonders vorteilhafte Ausgestaltungen und Weiterbildungen der Erfindung ergeben sich auch aus den abhängigen Ansprüchen sowie der nachfolgenden Beschreibung, wobei die unabhängigen Ansprüche einer Anspruchskategorie auch analog zu den abhängigen Ansprüchen und Ausführungsbeispielen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt ist die Ausgangsstellungs-Verriegelungseinrichtung derart ausgeführt, dass deren Umstellung zwischen der Verriegelungsstellung und der Funktionsstellung durch Verdrehung der Kolbeneinrichtung relativ zum Applikator-Gehäusekörper um eine Kolben-Längsachse erfolgt. Eine derartige Verriegelungseinrichtung lässt sich einfach und insbesondere intuitiv bedienen.

Vorzugsweise weist die Ausgangsstellungs-Verriegelungseinrichtung eine Rückdrehsicherung auf, die ein Rückverlagern der Ausgangsstellungs-Verriegelungseinrichtung von der Funktionsstellung in die Verriegelungsstellung verhindert. Eine solche Rückdrehsicherung führt zu einer Originalitätssicherung der Applikationsvorrichtung.

Die Ausgangsstellungs-Verriegelungseinrichtung ist bevorzugt derart ausgebildet, dass bei einem Umstellen der Ausgangsstellungs-Verriegelungseinrichtung von der Verriegelungsstellung in die Funktionsstellung, z. B. zwangsläufig, ein akustisches Signal abgegeben wird. Beispielsweise kann durch ein geeignetes Rastmittel oder dergleichen dafür gesorgt werden, dass beim Umstellen ein deutlich hörbares Klicken erfolgt.

Grundsätzlich könnte der Applikator-Gehäusekörper einteilig gefertigt sein. Bevorzugt ist der Applikator-Gehäusekörper jedoch mehrteilig, insbesondere zweiteilig, ausgebildet, besonders bevorzugt mit einem kanülenseitigen, d. h. mit der Kanüle gekoppelten, Implantat-Depotkörper, und einem Führungskörper. Im Implantat-Depotkörper befindet sich dabei vorzugsweise das Depotvolumen zur Bevorratung des Implantats. Der Führungskörper kann zum Führen der Verlagerungsbewegung der Kolbeneinrichtung zwischen der Ausgangsstellung und der Implantationsstellung dienen, d. h. der Kolben-Betätigungskörper ist im Führungskörper verschiebbar und geführt gelagert.

Der Implantat-Depotkörper ist bevorzugt an seinem rückwärtigen Ende, d. h. an dem von der Kanüle wegweisenden Ende, mittels Rastverbindungen mit dem Kolben-Bestätigungskörper gekoppelt.

Eine mehrteilige bzw. zweiteilige Ausbildung hat den Vorteil, dass die einzelnen Teile leichter zu fertigen sind, beispielsweise in einem Spritzgießverfahren. Außerdem wird hierdurch eine einfachere Einbringung des Implantats in das Depotvolumen ermöglicht, beispielsweise indem das Implantat vor der Kopplung des Implantat-Depotkörpers mit dem Kolben- Betätigungskörper von hinten in den Implantat-Depotkörper eingeschoben wird.

Wie später noch erwähnt wird, können so dem Implantat-Hersteller zwei getrennte Applikator-Baugruppen geliefert werden, und der Implantat-Hersteller kann in einfacher Weise für die Befüllung mit dem Implantat und die Endmontage sorgen.

Um für eine korrekte Montage der einzelnen Teile zu sorgen, können die zusammen zu montierenden Teile jeweils mit Passelementen, wie zum Beispiel Nuten und/oder Stegen oder dergleichen, versehen werden, sodass die Teile nur in genau einer Ausrichtung zusammenpassen.

In der Ausgangsstellung der Kolbeneinrichtung kann die Kanülenspitze vorzugsweise durch eine Schutzkappe abgedeckt sein, um eine Bedienperson vor Stichverletzungen durch die Kanüle zu sichern, solange die Applikationsvorrichtung nicht genutzt wird.

Die Schutzkappe kann zusätzlich eine unterstützende Funktion bei der Sicherung der Kolbeneinrichtung in der Ausgangsstellung haben, wenn die Schutzkappe so angeordnet ist, dass die Kolbeneinrichtung dann nicht nach vorne, d. h. in Richtung der Kanüle, geschoben werden kann.

Die Schutzkappe ist sinnvollerweise mit dem Applikator-Gehäusekörper, vorzugsweise mit einem Implantat-Depotkörper des Applikator-Gehäusekörpers, lösbar verbunden. Vorzugsweise erfolgt das Lösen der Schutzkappe vom Applikator-Gehäusekörper durch Verdrehung der Schutzkappe relativ zum Applikator-Gehäusekörper bzw. dem Implantat-Depotkörper um eine Kolben-Längsachse. Dies führt u. a. zu einem einfachen Aufbau der Applikationsvorrichtung.

Dabei ist die Applikationsvorrichtung vorzugsweise so aufgebaut, dass eine erste Drehrichtung, in der die Schutzkappe relativ zum Applikator-Gehäusekörper um die Kolben-Längsachse zu verdrehen ist, um die Schutzkappe vom Applikator-Gehäusekörper zu lösen, gleichgerichtet zu einer zweiten Drehrichtung ist, in der der Applikator-Gehäusekörper relativ zur Kolbeneinrichtung um die Kolben-Längsachse zu verdrehen ist, um die Ausgangsstellungs-Verriegelungseinrichtung von der Verriegelungsstellung in die Funktionsstellung umzustellen.

Durch diese Konstruktion kann das Risiko reduziert werden, dass der Nutzer irrtümlich die Verriegelung löst, anstatt die Schutzkappe abzuschrauben, wenn er aus Versehen die Schutzkappe in die falsche Richtung abschrauben möchte und dabei die Applikationsvorrichtung hinten an der Kolbeneinrichtung hält und die Schutzkappe relativ dazu verdreht. Wenn sich die Schutzkappe bei korrekter Drehrichtung leichter lösen lässt, als die Verdrehung von der Verriegelungsstellung in die Funktionsstellung möglich ist, ist sichergestellt, dass zuerst die Schutzkappe abgeschraubt wird. Bei einer falschen Drehrichtung bestünde ansonsten die Gefahr, dass der Applikator-Gehäusekörper mit der Schutzkappe relativ zur Kolbeneinrichtung mitverdreht wird und somit unbeabsichtigt eine Entriegelung erfolgt.

Die Applikationsvorrichtung weist vorzugsweise einen Nadelschutzkörper auf, der in der Ausgangsstellung der Kolbeneinrichtung in einer "Neutralstellung" angeordnet ist, in welcher die Kanüle herausragt (durch eine vordere, vom Kolben-Betätigungskörper wegweisende, Stirnseite des Nadelschutzkörpers, welche eine zentrale Öffnung aufweisen kann, durch die die Kanüle in der Neutralstellung hindurchgeht). In dieser "Neutralstellung" hat der Nadelschutzkörper keine Schutzwirkung, da die Kanülenspitze nicht durch den Nadelschutzkörper abgedeckt ist.

In der Neutralstellung des Nadelschutzkörpers kann sich dieser vorzugsweise auf Höhe des Implantat-Depotkörpers befinden, d. h. in der Ausgangsstellung kann der Nadelschutzkörper im Implantat-Depotkörper angeordnet sein.

Der Nadelschutzkörper ist so ausgebildet und an bzw. im Applikator-Gehäusekörper gelagert, dass er bei der Verlagerung der Kolbeneinrichtung von der Ausgangsstellung weiter, über die Implantationsstellung hinaus, in eine "Schutzstellung" überführt wird, in der der Nadelschutzkörper die freie Kanülenspitze aufnimmt. D. h. die Kolbeneinrichtung kann so ausgebildet sein, dass sie von der Implantationsstellung aus weiter nach vorne, zur Kanülenspitze geschoben wird und den Nadelschutzkörper dabei mitnimmt, so dass die vordere Stirnseite des Nadelschutzkörpers dann vor die Kanülenspitze verlagert ist und sich die Kanülenspitze folglich im Nadelschutzkörper befindet.

Ein Nadelschutzkörper hat sich zum Schutz des Anwenders vor Stichverletzungen bewährt. Über den Nadelschutzkörper kann zudem eine "Rückzugskraft" auf die Kanüle zum Entfernen dieser vom Implantations-Zielort ausgeübt werden. Tatsächlich wird dabei der Nadelschutzkörper mit seiner vorderen Stirnseite als Widerlager an der Oberfläche des Gewebes, in welchem das Implantat deponiert werden soll, z. B. der Haut eines Implantat-Empfängers, abgestützt und der Nadelschutzkörper wird dann mittels der Kolbeneinrichtung relativ zur Kanüle weiter nach vorne geschoben. Das kommt einem Rückzug der Kanüle in den Nadelschutzkörper und somit aus dem Gewebe, an dem der Nadelschutzkörper anliegt, gleich. Dabei bleibt das Implantat durch die parallele Bewegung von Nadelschutzkörper und Kolben automatisch an dem in der Implantationsstellung bereits erreichten Implantations-Zielort.

Vorzugsweise ist die Applikationsvorrichtung derart ausgebildet, dass das Kolben-Ausstoßende in der Schutzstellung des Nadelschutzkörpers in einem definierten Deponierungsabstand vor der vorderen Stirnseite des Nadelschutzkörpers liegt. Bevorzugt beträgt der Deponierungsabstand mindestens 4,0 mm, besonders bevorzugt mindestens 4,5 mm.

Der Deponierungsabstand bestimmt letztlich die Tiefe des Implantations-Zielorts unter der Gewebeoberfläche bzw. Hautoberfläche (also den kürzesten Abstand zwischen deponiertem Implantat und Oberfläche), wenn die Kanüle ordnungsgemäß bis zum Anschlag des Nadelschutzkörpers eingestochen wurde und der Kolben und der Nadelschutzkörper nach vorne geschoben werden, was einem Rückziehen der Nadel aus dem Körper entspricht.

Der Deponierungsabstand lässt sich durch die passende Wahl der Längen bzw. Längenverhältnisse der entsprechenden Teile der Applikationsvorrichtung, wie insbesondere der Kolbenstange, des Kolben-Betätigungskörpers und des Nadelschutzkörpers etc., in der gewünschten Weise einstellen.

Die Applikationsvorrichtung weist bevorzugt eine Schutzstellungs-Verriegelungseinrichtung zur Verriegelung der Kolbeneinrichtung am Applikator-Gehäusekörper, vorzugsweise am Implantat-Depotkörper, nach Erreichen der Schutzstellung auf.

Eine solche Schutzstellungs-Verriegelungseinrichtung kann verhindern, dass die Kolbeneinrichtung unerwünscht die einmal erreichte Schutzstellung verlässt, und sorgt somit für eine sichere Funktion der Applikationsvorrichtung und insbesondere für eine sichere Wirkung des Nadelschutzkörpers.

Wie bereits erwähnt, kann die Applikationsvorrichtung bevorzugt zumindest zwei zunächst getrennte und, vorzugsweise über eine Rastverbindung, miteinander verbindbare Applikator-Baugruppen aufweisen.

Dabei kann vorzugsweise die eine der beiden Applikator-Baugruppen als Depot-Baugruppe ausgeführt sein und die Kanüle und den Implantat-Depotkörper aufweisen. Die andere der beiden Applikator-Baugruppen kann hingegen vorzugsweise als Kolben-Baugruppe ausgeführt sein und die Kolbeneinrichtung und den Führungskörper aufweisen.

Eine derartige Gestaltung der Applikationsvorrichtung ermöglicht es, die beiden Baugruppen zunächst getrennt voneinander herzustellen und nach Einsetzen des Implantats endzumontieren.

Grundsätzlich könnte aber eine Trennung in Baugruppen auch an einer anderen Stelle verlaufen. Z. B. könnten der Implantat-Depotkörper oder die Kolben-Baugruppe selbst zweiteilig ausgebildet sein.

Besonders bevorzugt können dabei die beiden Baugruppen so ausgebildet sein, dass sie ausschließlich gerichtet und nicht versehentlich um 180° gedreht montiert werden können. Dies ist z. B. vorzugsweise mit Hilfe einer Nut an einer der Baugruppen und einem dazu passenden Steg an der anderen Baugruppe realisierbar.

Andere Verbindungen der Applikator-Baugruppen sind auch möglich, z. B. mittels einer Klebeverbindung.

Bevorzugt kann die Depot-Baugruppe außerdem die bereits erwähnte Schutzkappe und/oder den Nadelschutzkörper aufweisen. Diese Gestaltungen der Depot-Baugruppe haben sich zum Erreichen einer effizienten Montierbarkeit bewährt.

Ein Ausführungsbeispiel der Erfindung wird nachfolgend anhand der Zeichnungen näher erläutert. Diese zeigen:
- Fig. 1: eine Explosionsdarstellung einer Applikationsvorrichtung für mindestens ein Implantat;
- Fig. 2: einen Axialschnitt durch einen kanülenseitigen Abschnitt der fertig montierten Applikationsvorrichtung in einer Ausgangsstellung;
- Fig. 3: perspektivisch eine Schutzkappe der Applikationsvorrichtung;
- Fig. 4: perspektivisch eine Kanüle sowie einen kanülenseitigen Abschnitt eines Implantat-Depotkörpers der Applikationsvorrichtung;
- Fig. 5: perspektivisch einen Applikator-Gehäusekörper der Applikationsvorrichtung;
- Fig. 6: eine Kolbeneinrichtung der Applikationsvorrichtung mit einem Kolben und einem Kolben-Betätigungskörper, gesehen aus einer ersten Blickrichtung;
- Fig. 7: die Kolbeneinrichtung nach Fig. 6, gesehen aus einer zweiten Blickrichtung, bei der die Kolbeneinrichtung im Vergleich zu Fig. 6 etwa um 90° um eine Kolben-Längsachse verdreht ist;
- Fig. 8: einen Längsschnitt durch die Applikationsvorrichtung nach Fig. 1 in einer Ausgangsstellung der Kolbeneinrichtung, wobei die Kanüle der Applikationsvorrichtung zur Applikation des Implantats bereits in das Gewebe eingesteckt ist;
- Fig. 9: einen Längsschnitt durch die Applikationsvorrichtung wie in Fig. 8, jedoch in der Implantationsstellung, wobei der Kolbenbetätigungskörper an den Nadelschutzkörper anschlägt;
- Fig. 10: einen Längsschnitt durch die Applikationsvorrichtung wie in Fig. 8 oder 9, jedoch in der Schutzstellung des Nadelschutzkörpers mit weiter vorgeschobenem Kolben, so dass das Kolben-Ausstoßende außerhalb der Kanülenspitze und noch im Gewebe liegt;
- Fig. 11: einen Längsschnitt durch die Applikationsvorrichtung wie in Fig. 10, wobei der Kolben aus dem Gewebe herausgezogen ist.

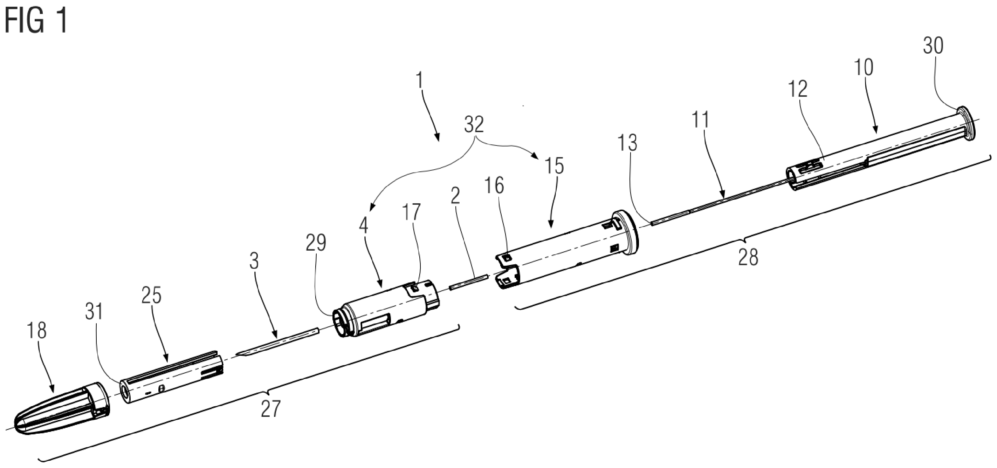

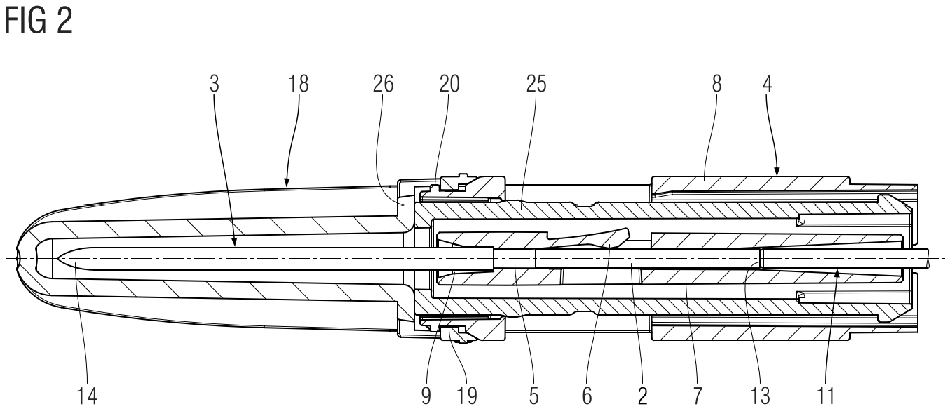

Eine Applikationsvorrichtung 1 dient zur Implantation mindestens eines Implantats 2 (vgl. Fig. 1 und 2).

Hauptkomponenten der Applikationsvorrichtung 1 sind in der Explosionsdarstellung der Fig. 1 gezeigt.

Die Applikationsvorrichtung 1 hat eine Kanüle 3, über die das Implantat 2 einem Implantations-Zielort, beispielsweise im subkutanen Bauchgewebe eines Patienten, zugeführt wird. Die Kanüle 3 ist aus Edelstahl. Die Kunststoffkomponenten der Applikationsvorrichtung 1 können als Spritzgusskomponenten gefertigt sein.

Die Applikationsvorrichtung 1 hat weiterhin einen Implantat-Depotkörper 4 mit einem Depotvolumen 5 (vgl. Fig. 2) zur Bevorratung des Implantats 2. Der Implantat-Depotkörper 4 ist aus Kunststoffmaterial und kann beispielsweise aus Methylmethacrylat-Butadien-Styrol (MBS) gefertigt sein. Zur Fixierung des Implantats 2 im Depotvolumen 5 hat der Implantat-Depotkörper 4 ein Fixierelement 6 in Form einer federnden Nase. Diese Nase 6 sorgt, wenn das Implantat 2 eine Zielposition im Depotvolumen 5 erreicht hat, für eine reib- und/oder formschlüssige Fixierung des Implantats 2 im Depotvolumen 5. Alternativ oder zusätzlich kann das Fixierelement 6 eine Klemmung des Implantats 2 herbeiführen.

Das Depotvolumen 5 ist gebildet durch einen inneren Hülsenabschnitt 7 des Implantat-Depotkörpers 4. Dieser innere Hülsenabschnitt 7 steht über einen in der Zeichnung nicht näher ersichtlichen, radial verlaufenden Steg mit einem äußeren Hülsenabschnitt 8 des Implantat-Depotkörpers 4 in Verbindung. Die beiden Hülsenabschnitte 7, 8 sind jeweils einstückige, integrale Bestandteile des Implantat-Depotkörpers 4, der durch Spritzguss hergestellt sein kann.

Kanülenseitig hat der innere Hülsenabschnitt 7 einen konisch zulaufenden Kanülen-Aufnahmebereich 9 zur Halterung eines implantatseitigen Endabschnitts der Kanüle 3. Über den Kanülen-Aufnahmebereich 9 ist die Kanüle 3 an dem inneren Hülsenabschnitt 7 fixiert, vorzugsweise verklebt oder verpresst. Das Depotvolumen 5 fluchtet mit einem Lumen der Kanüle 3.

Die Applikationsvorrichtung 1 hat weiterhin eine Kolbeneinrichtung 10 mit einem Kolben 11 und mit einem Kolben-Betätigungskörper 12. Der Kolben-Betätigungskörper 12 ist mit dem Kolben 11 fest verbunden. Der Kolben 11 ist in diesem Fall aus Edelstahl gefertigt. Der Kolben-Betätigungskörper 12 ist aus Kunststoff, beispielsweise aus Polybutylenterephthalat (PBT), gefertigt.

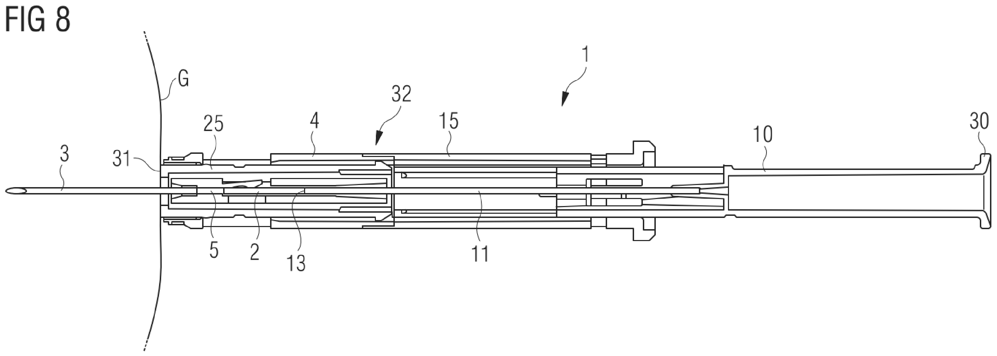

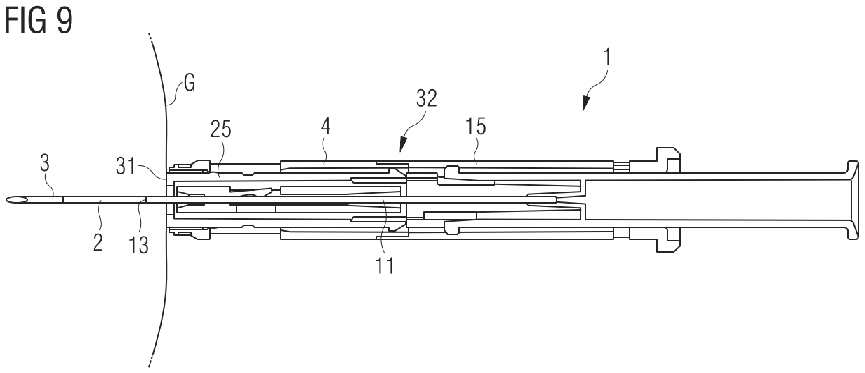

Der Kolben 11 hat einen Außendurchmesser, der an einen Innendurchmesser einerseits des Depotvolumens 5 und andererseits des Lumens der Kanüle 3 angepasst ist. Die gesamte Kolbeneinrichtung 10 und somit der Kolben 11 ist verlagerbar zwischen einer Ausgangsstellung und einer Implantationsstellung. In der Ausgangsstellung des Kolbens 11 liegt das Depotvolumen 5 zwischen dem Kolben 11 und dem Endabschnitt der Kanüle 3 (siehe auch Figur 8). In der Implantationsstellung liegt ein Kolben-Ausstoßende 13 im Bereich einer freien Kanülenspitze 14 der Kanüle 3, wobei das Kolben-Ausstoßende 13 zumindest so weit nach vorne in die Kanüle 3 verlagert ist, dass das Implantat in der Kanüle positioniert ist, und zwar schon am Implantations-Zielort (aber noch von der Kanüle 3 umgeben), wenn die Kanüle 3 ordnungsgemäß in das Gewebe G eingestochen ist (siehe Figur 9).

Weiterhin umfasst die Applikationsvorrichtung 1 einen Applikator-Gehäusekörper 32 mit einem Führungskörper 15 zum Führen einer Verlagerungsbewegung der Kolbeneinrichtung 10 und insbesondere des Kolben-Betätigungskörpers 12 zwischen der Ausgangsstellung und der Implantationsstellung der Kolbeneinrichtung 10. Der Applikator-Gehäusekörper 32 und insbesondere der Führungskörper 15 ist bevorzugt aus Kunststoff gefertigt, insbesondere aus MBS.

Über eine Rastverbindung mit Rastöffnungen 16 im Führungskörper 15 und Rasthaken 17 am Implantat-Depotkörper 4 ist bei vollständig montierter Applikationsvorrichtung 1 der Führungskörper 15 mit dem Implantat-Depotkörper 4 unter Bildung des Applikator-Gehäusekörpers 32 fest und insbesondere unlösbar miteinander verbunden. Diese Verbindung des Führungskörpers 15 mit dem Implantat-Depotkörper 4 ist über Nut und Stege verdrehsicher ausgeführt, sodass insbesondere eine Falschmontage dieser beiden Komponenten (z. B. um 180° verdreht) miteinander vermieden wird. Entsprechende Nuten und Stege sind in einer Hülsen-Profilierung des Implantat-Depotkörpers 4 einerseits und des Führungskörpers 15 andererseits zur Verdrehsicherung dieser beiden Komponenten zueinander vorgesehen.

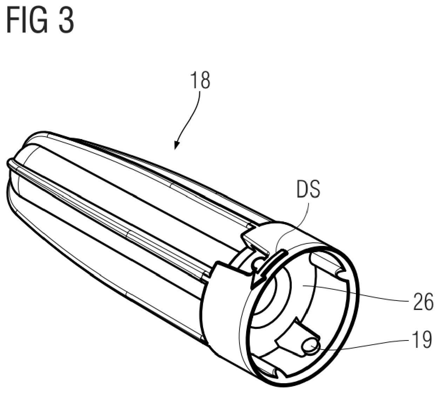

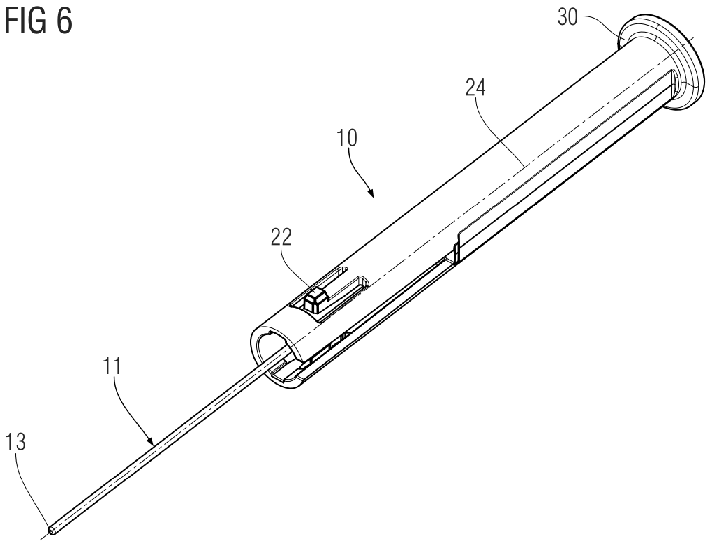

Weiterhin hat die Applikationsvorrichtung 1 eine Schutzkappe 18 zur Abdeckung der Kanülenspitze 14 sowie eines weiteren freiliegenden Abschnitts der Kanüle 3 in der Ausgangsstellung der Kolbeneinrichtung 10. Über eine Rastverbindung, die nach Art einer Bajonettverbindung ausgebildet ist, ist die Schutzkappe 18 lösbar mit dem Implantat-Depotkörper 4 verbunden, wobei die Schutzkappe 18 relativ zum Implantat-Depotkörper 4 um die Kolben-Längsachse 24 in einer ersten Drehrichtung DS verdreht wird (siehe Figuren 3 und 6). Die Schutzkappe 18 weist hierzu Rastnasen 19 auf, die mit einer Bajonett-Kulisse 20 zusammenwirken, die außen in dem äußeren Hülsenabschnitt des Implantat-Depotkörpers 4 in einem kanülenseitigen Bereich von diesem ausgeführt ist.

Die Schutzkappe 18 ist aus Kunststoff gefertigt, insbesondere aus Polypropylen (PP).

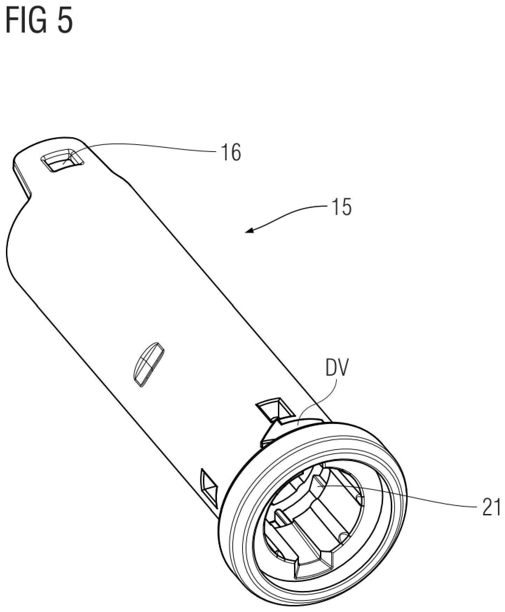

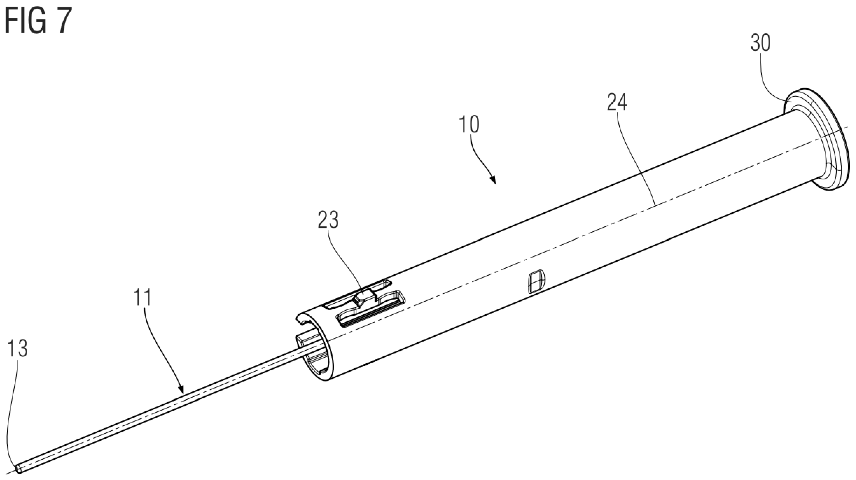

Die Applikationsvorrichtung 1 hat weiterhin eine Ausgangsstellungs-Verriegelungseinrichtung 21 bis 23 zur überwindbaren Verriegelung des Applikator-Gehäusekörpers 32, hier des Führungskörpers 15, mit der Kolbeneinrichtung 10 in deren Ausgangsstellung. Diese Ausgangsstellungs-Verriegelungseinrichtung weist gehäusekörperseitige Verriegelungseinheiten in Form von Rasthaken 21 auf, die innenliegend im hülsenförmigen Applikator-Gehäusekörper 15 angeordnet sind (vgl. Fig. 5). Weiterhin hat die Ausgangsstellungs-Verriegelungsstellungseinrichtung kolbenseitige Verriegelungselemente in Form eines ersten Typs Rastnase 22 (vgl. Fig. 6) und eines zweiten Typs Rastnase 23 (vgl. Fig. 7).

Die Ausgangsstellungs-Verriegelungseinrichtung 21 bis 23 ist umstellbar zwischen einer Verriegelungsstellung, in der die Kolbeneinrichtung 10 am Applikator-Gehäusekörper 32 bzw. am Führungskörper 15 in der Ausgangsstellung fixiert ist, und einer Funktionsstellung, in der die Kolbeneinrichtung 10 zur Verlagerung des Kolbens 11 relativ zum Applikator-Gehäusekörper 32 bzw. Führungskörper 15 verlagerbar ist. Diese Umstellung der Ausgangsstellungs-Verriegelungseinrichtung 21 bis 23 zwischen der Verriegelungsstellung und der Funktionsstellung erfolgt durch Verdrehung der Kolbeneinrichtung 10 relativ zum Applikator-Gehäusekörper 15 um eine Kolben-Längsachse 24 (vgl. z. B. Fig. 6) in eine zweite Drehrichtung DV (siehe Fig. 5). Diese beiden Stellungen sind in den Fig. 6 und 7 dargestellt. Der Rasthaken 21 im Applikator-Gehäusekörper 15 dient weiterhin als Rückdrehsicherung der Ausgangsstellungs-Verriegelungseinrichtung 21 bis 23. Diese Rückdrehsicherung 21 verhindert ein Rückverlagern der Ausgangsstellungs-Verriegelungseinrichtung 21 bis 23 von der Funktionsstellung zurück in die Verriegelungsstellung.

Die Rastnase 23 des zweiten Typs, die in eine entsprechende Nut im Applikator-Gehäusekörper 32 bzw. Führungskörper 15 einrastet, stellt ein Teil der Verriegelung durch die Ausgangsstellungs-Verriegelungseinrichtung 21 bis 23 in der Verriegelungsstellung dar. In der Funktionsstellung rasten die Rastnasen 23 des Typs zwei hinter die Rasthaken 21, die ein Rückdrehen in die Verriegelungsstellung verhindern.

Weiterhin hat die Applikationsvorrichtung 1 einen Nadelschutzkörper 25. Dieser ist in der Ausgangsstellung der Kolbeneinrichtung 10 in einer Neutralstellung auf Höhe des Implantat-Depotkörpers 4 angeordnet (vgl. Fig. 2). Der Nadelschutzkörper 25 hat eine Hülsen-Grundform und ist in der Neutralstellung zwischen dem inneren Hülsenabschnitt 7 und dem äußeren Hülsenabschnitt 8 des Implantat-Depotkörpers 4 angeordnet. Der Verbindungssteg zwischen den beiden Hülsenabschnitten 7, 8 durchdringt einen entsprechenden Längsschlitz im Nadelschutzkörper 25, der in der Zeichnung nicht gezeigt ist und eine Axialverlagerung des Nadelschutzkörpers 25 relativ zum Implantat-Depotkörper 4 erlaubt. Bei aufgesteckter Schutzkappe 18 ist der Nadelschutzkörper 25 in der Neutralstellung über Anlage an einem Sicherungsbund 26 der Schutzkappe 18 gesichert.

Der Nadelschutzkörper 25 ist aus Kunststoffmaterial, insbesondere aus PBT, gefertigt.

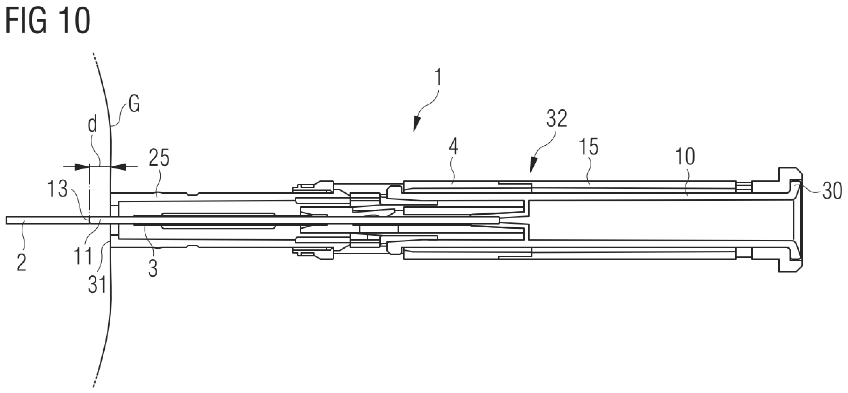

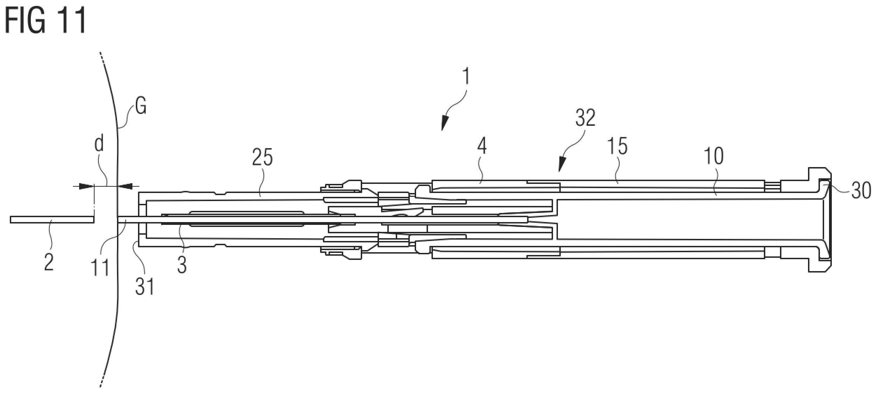

Bei Verlagerung der Kolbeneinrichtung 10 in Richtung der Kanülenspitze 14 über die Implantationsstellung hinaus wird der Nadelschutzkörper 25 vom Kolben-Betätigungskörper 12 mitgenommen und in eine Schutzstellung überführt (siehe Fig. 10 und 11). In dieser Schutzstellung ist der Nadelschutzkörper 25 bis hin zur freien Kanülenspitze 14 der Kanüle 3 verlagert (d. h. die vordere Stirnseite 31 des Nadelschutzkörpers liegt vor der Kanülenspitze 14) und deckt somit die Kanüle 3 zum Schutz vor Verletzungen, insbesondere an der Kanülenspitze 14 ab. In dieser Stellung kann wie dargestellt auch der Kolben 11 vorne aus der Kanüle 3 heraus ragen.

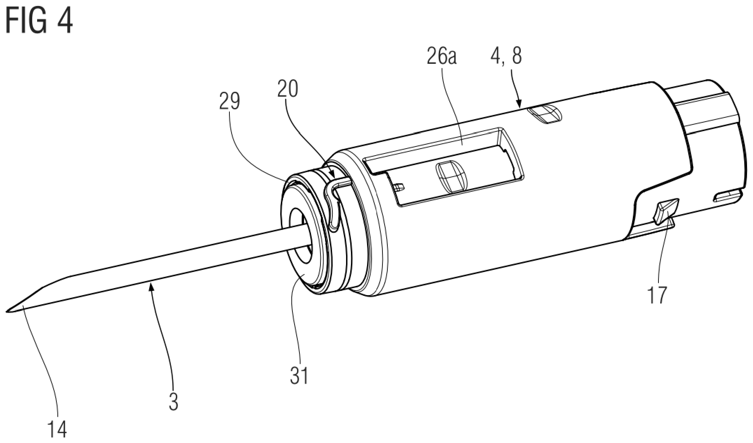

Weiterhin hat die Applikationsvorrichtung 1 eine Schutzstellungs-Verriegelungseinrichtung zur Verriegelung der Kolbeneinrichtung 10 am Implantat-Depotkörper 4, wenn der Nadelschutzkörper 25 seine Schutzstellung erreicht hat. Teil dieser Schutzstellungs-Verriegelungseinrichtung kann die oben bereits erwähnte Rastnase 22 des ersten Typs sein (vgl. Fig. 6). Zu dieser Schutzstellungs-Verriegelungseinrichtung gehört weiterhin ein Rastfenster 26a (vgl. Fig. 4), das im äußeren Hülsenabschnitt 8 des Implantat-Depotkörpers 4 ausgeführt ist.

Die Applikationsvorrichtung 1 hat vor deren Endmontage zwei zunächst getrennte und über die Rastverbindung 16, 17 miteinander verbindbare Applikator-Baugruppen, nämlich eine Depot-Baugruppe 27 und eine Kolben-Baugruppe 28 (vgl. Fig. 1). Die Depot-Baugruppe 27 umfasst die Kanüle 3 und den Implantat-Depotkörper 4. Weiterhin umfasst die Depot-Baugruppe 27 die Schutzkappe 18 und den Nadelschutzkörper 25. Die Kolben-Baugruppe 28 umfasst die Kolbeneinrichtung 10 und den Führungskörper 15.

Vor der Endmontage der Applikationsvorrichtung 1 wird das Wirkstoff-Implantat 2 in das Depotvolumen 5 eingeschoben. Anschließend werden die beiden Baugruppen 27, 28 bei der Endmontage zusammengesteckt und sind dann über die Rastverbindung 16, 17 fest miteinander verbunden. Die fertig montierte Applikationsvorrichtung 1 könnte dann z. B. sterilisiert und in eine sterile Verpackung eingesiegelt werden. Ebenso könnte das gesamte System (Applikationsvorrichtung mit Verpackungsbeutel) zur Sterilisierung end-bestrahlt werden, beispielsweise mit Gamma- oder Röntgen-Bestrahlung. Theoretisch kann die Applikationsvorrichtung aber auch für nicht-sterile Zwecke verwendet werden.

Je nach Ausführung der Applikationsvorrichtung 1 können verschiedene Größen, insbesondere verschiedene Durchmesser des Depotvolumens 5 zur Aufnahme verschieden großer Implantate 2 bereitgestellt werden. Somit können mit grundsätzlich gleich aufgebauten Applikationsvorrichtungen nach Art der Applikationsvorrichtung 1 verschiedene Implantatgrö-ßen verabreicht werden.

In der Ausgangsstellung liegt zudem einerseits der Kolben-Betätigungskörper 12 axial am Nadelschutzkörper 25 und dieser wiederum axial an einem Sicherungsbund 26 der Schutzkappe 18 an, so dass die Schutzkappe 18 ein Auslösen der Applikationsvorrichtung 1, also ein Überführen der Kolbeneinrichtung 10 von der Ausgangsstellung in die Implantationsstellung, verhindert (vgl. Fig. 1 und 2).

Bei der Anwendung der Applikationsvorrichtung 1 wird zunächst die Schutzkappe 18 vom Implantat-Depotkörper 4 durch Überwinden der Bajonettverbindung 19, 20 gelöst. Anschließend wird der Kolben-Betätigungskörper 12 und somit die gesamte Kolbeneinrichtung 10 von der Verriegelungsstellung in die Funktionsstellung der Ausgangsstellungs-Verriegelungseinrichtung 21 bis 23 verdreht.

Um zu verhindern, dass bei einem Versuch, die Schutzkappe in die falsche Richtung zu lösen, aus Versehen die Ausgangsstellungs-Verriegelungseinrichtung 21 bis 23 gelöst wird, ist die erste Drehrichtung DS (siehe Fig. 3), in der die Schutzkappe 18 zum Lösen relativ zum Implantat-Depotkörpers 4 um die Kolben-Längsachse 24 zu verdrehen ist, gleichgerichtet zu der zweiten Drehrichtung DV (siehe Fig. 5), in der der Applikator-Gehäusekörper 15 relativ zur Kolbeneinrichtung 10 zu verdrehen ist, um die Ausgangsstellungs-Verriegelungseinrichtung 21 bis 23 von der Verriegelungsstellung in die Funktionsstellung umzustellen.

Bei Anwendung des Applikators wird die Kanüle 3 bis zu dem durch eine Stirnseite 29 des Implantat-Depotkörpers 4 und/oder die vordere Stirnseite 31 des Nadelschutzkörpers 25 gebildeten Anschlag unter die Haut des Patienten G gesetzt (siehe Fig. 8).

Nun wird ein Betätigungsende 30 des Kolben-Betätigungskörpers 12 beispielsweise mit dem Daumen eines Anwenders betätigt. Die Kolbeneinrichtung 10 wird dadurch durch den Applikator-Gehäusekörper 15 in Richtung der Kanüle 3 in die Implantationsstellung geschoben. Der Kolben 11 schiebt hierbei das Implantat 2 aus dem Depotvolumen 5 durch die Kanüle 3 in eine Position, welche dem gewünschten Implantations-Zielort entspricht, wobei jedoch das Implantat 2 noch von der Kanüle 3 umgeben ist. Diese Implantationsstellung ist in Figur 9 gezeigt.

In der Implantationsstellung stößt der Kolbenbetätigungskörper 12 mit seinem vorderen Ende am hinteren Ende des Nadelschutzkörpers 25 an, so dass dann bei einem weiteren Verschieben der Kolbeneinrichtung 10 nach vorne, d. h. über die Implantationsstellung hinaus, der Nadelschutzkörper 25 parallel zum Kolben 11 nach vorne über die Kanüle 3 geschoben wird. Eine vordere Stirnseite 31 übernimmt dabei die Anschlagfunktion an der Haut G des Patienten um die Kanülen-Einstichstelle herum. Auf diese Weise ergibt sich eine Art Rückzug der Kanüle 3 aus der Haut G in den Nadelschutzkörper, wobei gleichzeitig mit Hilfe des Kolbens 11 gewährleistet wird, dass das Implantat 2 im Wesentlichen am Implantations-Zielort unter der Haut G verbleibt. Dies ist in Figur 10 gezeigt.

Eventuell kann das Implantat 2 zu Beginn des Herausziehens der Kanüle 3 noch ein wenig innerhalb der Kanüle 3 nach vorne geschoben werden (so dass sich der Implantations-Zielort noch ein wenig verschieben kann), je nachdem, wie stark sich die vordere Stirnseite 31 des Nadelschutzkörpers 25 noch in das Gewebe G eindrücken lässt. Wie aus Figur 10 gut zu erkennen ist, bestimmt letztlich der Deponierungsabstand d zwischen dem Kolben-Ausstoßende 13 und der vorderen Stirnseite 31 des Nadelschutzkörpers 25 in der Schutzstellung die Tiefe des Implantations-Zielorts unter der Hautoberfläche.

In dieser Schutzstellung des Nadelschutzkörpers 25 und der Kolbeneinrichtung 10 rastet hier die Kolbeneinrichtung 10 über die Schutzstellungs-Verriegelungseinrichtung am Implantat-Depotkörper 4 ein, so dass bevorzugt ein Rückstellen der Kolbeneinrichtung 10 aus der Schutzstellung zurück in die Ausgangsstellung nicht mehr möglich ist. In der Schutzstellung ist der Nadelschutzkörper 25 vollständig über die Kanüle 3 geschoben und schützt den Anwender vor Verletzungen, wobei jedoch der Kolben 11 bereits vorne aus der Kanüle 3 herausragt.

Die Applikation ist abgeschlossen, wenn die Kanüle 3 vollständig aus der Haut G entfernt ist und dabei der Kolben 11 das Implantat 2 vollständig aus der Kanüle 3 ausgeschoben hat und auch der Kolben 11 aus der Haut G gezogen wurde. Dies ist in Figur 11 gezeigt. Das Implantat verbleibt dann sicher am Implantations-Zielort, im Wesentlichen im Deponierungsabstand d, unter der Hautoberfläche.

Vorstehend wurde die Applikation genau eines Implantats mit der Applikationsvorrichtung beschrieben. Grundsätzlich ist es jedoch auch möglich, die Applikationsvorrichtung 1 so auszuführen, dass hierüber mehrere Implantate appliziert werden können, z. B. in einer Art "Kette" von mehreren (vorzugsweise kürzeren) Implantaten hintereinander.

## Patentansprüche

1. Applikationsvorrichtung (1) für mindestens ein Implantat (2)
- mit einer Kanüle (3), über die das Implantat (2) einem Implantations-Zielort zugeführt wird,
- mit einem Applikator-Gehäusekörper (32) mit einem Depotvolumen (5) zur Bevorratung des Implantats (2),
- mit einer Kolbeneinrichtung (10) mit einem Kolben (11), der verlagerbar ist zwischen
-- einer Ausgangsstellung, in der ein Kolben-Ausstoßende (13) des Kolbens (11) auf einer von einer freien Kanülenspitze (14) abgewandten Seite des Depotvolumens (5) liegt, und
-- einer Implantationsstellung, in der ein Kolben-Ausstoßende (13) im Bereich einer freien Kanülenspitze (14) liegt,
-- wobei die Kolbeneinrichtung (10) einen Kolben-Betätigungskörper (12) aufweist, mit dem der Kolben (11), vorzugsweise fest, verbunden ist,
- wobei der Applikator-Gehäusekörper (32) zum Führen einer Verlagerungsbewegung der Kolbeneinrichtung (10) zwischen der Ausgangsstellung und der Implantationsstellung ausgebildet ist,
- mit einer Ausgangsstellungs-Verriegelungseinrichtung (21 bis 23) zur überwindbaren Verriegelung des Applikator-Gehäusekörpers (32) mit der Kolbeneinrichtung (10) in deren Ausgangsstellung, wobei die Ausgangsstellungs-Verriegelungseinrichtung (21 bis 23) umstellbar ist zwischen
-- einer Verriegelungsstellung, in der die Kolbeneinrichtung (10) am Applikator-Gehäusekörper (32) in der Ausgangsstellung fixiert ist, und
-- einer Funktionsstellung, in der die Kolbeneinrichtung (10) zur Verlagerung des Kolbens (11) relativ zum Applikator-Gehäusekörper (32) verlagerbar ist.

2. Applikationsvorrichtung nach Anspruch 1, **dadurch gekennzeichnet, dass** die Ausgangsstellungs-Verriegelungseinrichtung (21 bis 23) derart ausgeführt ist, dass deren Umstellung zwischen der Verriegelungsstellung und der Funktionsstellung durch Verdrehung der Kolbeneinrichtung (10) relativ zum Applikator-Gehäusekörper (32) um eine Kolben-Längsachse (24) erfolgt.

3. Applikationsvorrichtung nach Anspruch 1 oder 2, **dadurch gekennzeichnet, dass** die Ausgangsstellungs-Verriegelungseinrichtung (21 bis 23) eine Rückdrehsicherung (21) aufweist, die ein Rückverlagern der Ausgangsstellungs-Verriegelungseinrichtung (21 bis 23) von der Funktionsstellung in die Verriegelungsstellung verhindert.

4. Applikationsvorrichtung nach einem der vorstehenden Ansprüche, **dadurch gekennzeichnet, dass** die Ausgangsstellungs-Verriegelungseinrichtung (21 bis 23) derart ausgebildet ist, dass bei einem Umstellen der Ausgangsstellungs-Verriegelungseinrichtung (21 bis 23) von der Verriegelungsstellung in die Funktionsstellung ein akustisches Signal abgegeben wird.

5. Applikationsvorrichtung nach einem der vorstehenden Ansprüche, **dadurch gekennzeichnet, dass** der Applikator-Gehäusekörper (32) zweiteilig ausgebildet ist, mit einem kanülenseitigen Implantat-Depotkörper (4), in welchem sich vorzugsweise das Depotvolumen (5) zur Bevorratung des Implantats (2) befindet, und einem Führungskörper (15) zum Führen der Verlagerungsbewegung der Kolbeneinrichtung (10) zwischen der Ausgangsstellung und der Implantationsstellung.

6. Applikationsvorrichtung nach einem der vorstehenden Ansprüche, **gekennzeichnet durch** eine Schutzkappe (18) zur Abdeckung der Kanülenspitze (14) in der Ausgangsstellung der Kolbeneinrichtung (10).

7. Applikationsvorrichtung nach Anspruch 6, **dadurch gekennzeichnet, dass** die Schutzkappe (18) mit dem Applikator-Gehäusekörper (32) lösbar verbunden (19, 20) ist, so dass vorzugsweise ein Lösen der Schutzkappe (18) vom Applikator-Gehäusekörper (32) durch Verdrehung der Schutzkappe (18) relativ zum Applikator-Gehäusekörper (32) um eine Kolben-Längsachse (24) erfolgt.

8. Applikationsvorrichtung nach Anspruch 7, **dadurch gekennzeichnet, dass** eine erste Drehrichtung (DS), in der die Schutzkappe (18) relativ zum Applikator-Gehäusekörper (32) um die Kolben-Längsachse (24) zu verdrehen ist, um die Schutzkappe (18) vom Applikator-Gehäusekörper (32) zu lösen, gleichgerichtet zu einer zweiten Drehrichtung (DV) ist, in der der Applikator-Gehäusekörper (32) relativ zur Kolbeneinrichtung (10) um die Kolben-Längsachse (24) zu verdrehen ist, um die Ausgangsstellungs-Verriegelungseinrichtung (21 bis 23) von der Verriegelungsstellung in die Funktionsstellung umzustellen.

9. Applikationsvorrichtung nach einem der vorstehenden Ansprüche, **gekennzeichnet durch** einen Nadelschutzkörper (25), der in der Ausgangsstellung der Kolbeneinrichtung (10) in einer Neutralstellung angeordnet ist, in der die Kanüle (3) aus dem Nadelschutzkörper (25) herausragt, und der bei der Verlagerung der Kolbeneinrichtung (10) von der Ausgangsstellung über die Implantationsstellung hinaus in eine Schutzstellung überführt ist, in der der Nadelschutzkörper (25) die freie Kanülenspitze (14) aufnimmt.

10. Applikationsvorrichtung nach Anspruch 9, **dadurch gekennzeichnet, dass** die Applikationsvorrichtung derart ausgebildet ist, dass das Kolben-Ausstoßende (13) in der Schutzstellung des Nadelschutzkörpers (25) in einem definierten Deponierungsabstand (d) vor einer vorderen Stirnseite (31) des Nadelschutzkörpers (25) liegt.

11. Applikationsvorrichtung nach Anspruch 10, **dadurch gekennzeichnet, dass** der Deponierungsabstand (d) mindestens 4,0 mm, bevorzugt mindestens 4,5 mm, beträgt.

12. Applikationsvorrichtung nach einem der vorstehenden Ansprüche 9 bis 11, **gekennzeichnet durch** eine Schutzstellungs-Verriegelungseinrichtung zur Verriegelung der Kolbeneinrichtung (10) am Applikator-Gehäusekörper (32) nach Erreichen der Schutzstellung.

13. Applikationsvorrichtung nach einem der vorstehenden Ansprüche, **gekennzeichnet durch** zwei zunächst getrennte und über eine Rastverbindung (16, 17) miteinander verbindbare Applikator-Baugruppen (27, 28),
- wobei vorzugsweise die eine der beiden Applikator-Baugruppen als Depot-Baugruppe (27) ausgeführt ist und die Kanüle (3) und den Implantat-Depotkörper (4) aufweist,
- wobei vorzugsweise die andere der beiden Applikator-Baugruppen als Kolben-Baugruppe (28) ausgeführt ist und die Kolbeneinrichtung (10) und den Führungskörper (15) aufweist.

14. Applikationsvorrichtung nach Anspruch 13, **dadurch gekennzeichnet, dass** die Depot-Baugruppe (27) die Schutzkappe (18) und/oder den Nadelschutzkörper (25) aufweist.

15. Applikationsvorrichtung nach einem der vorstehenden Ansprüche zur humanmedizinischen, veterinärmedizinischen oder kosmetischen Anwendung.

16. Applikationsvorrichtung zur Verwendung bei der Verabreichung von Arzneistoffen, bevorzugt von (synthetischen) Analoga des Neurohormons Gonadotropin-Releasing-Hormon (GnRH), besonders bevorzugt zur Behandlung von Prostata- oder Brustkrebs, Endometriose, Uterusmyom, Endometriumablation, verfrüht einsetzender Pubertät (Pubertas praecox), Transsexualität, Cluster-Kopfschmerz und in der humanmedizinischen und veterinärmedizinischen Reproduktionsmedizin.
